# EUROPEAN PATENT APPLICATION

(11) **EP 2 522 713 A2**
(43) Date of publication of application: **14.11.2012**
(21) Application number: 12165142.6
(22) Date of filing: 23.04.2012
(51) Int. Cl.: C11C 3/10

(54) **A method of producing biodiesel using heterogeneous catalytic system**

(30) Priority: 20.04.2011 MY PI2011001762
(71) Applicant: Universiti Putra Malaysia, Selangor (MY)
(72) Inventor: YAP, Taufiq Yun Hin, 43400 Selangor Darul Ehsan (MY)
(74) Representative: KIPA AB

(57) **Abstract**

A method of producing a mixture of methyl ester from an oil source comprises the step of reacting the oil source with an alcohol in the presence of a heterogeneous catalytic system derived from mixed metal oxide of calcium and a second metal.

## Description

### FIELD OF INVENTION

The present invention relates to a method of producing biodiesel or methyl ester from an oil source containing relatively high content of free fatty acid (FFA). More specifically, the disclosed method employs an unique catalytic system, which can tolerate in the existence of high content of FFA, to produce methyl ester without requiring pre-treatment to remove the FFA from the oil source.

### BACKGROUND OF THE INVENTION

Biodiesel has become very attractive as an alternative biomass-based fuel because of its environmental advantages - it is non-toxic, biodegradable and has lower emission of CO, unburned hydrocarbon, particulate matter and SO₂ Further, high energetic efficiency of biodiesel has rendered it the ideal candidate to partly substitute use of fossil fuel in certain area. Chemically, biodiesel is a mixture of methyl esters with long-chain fatty acids (chain length C14-C22) and is typically made from biological resources such as vegetable oils, seeds, animal fats or used frying oils.

Conventional manufacturing operation for biodiesel production involves catalyzed transesterification reaction which reacts triglycerides in oils or fats with short chain alcohol (methanol or ethanol) under the influence of catalyst. Conventional operation usually uses a strong basic (e.g., NaOH or KOH) solution as a homogeneous catalyst and food-grade vegetable oils (canola, sunflower, rapeseed and palm oil) as the raw material. Many problems have been raised from such conventional process. Particularly, using homogeneous catalysts limits the operation in batch-mode processing followed by a catalyst separation step. Moreover, these catalysts are soluble in the end products of the transesterification process that physically removing the used catalysts via filtration is infeasible. Both fatty acid methyl esters and glycerin phases produced have to be neutralized by mineral acids or bases and washed with water. This post-reaction treatment in removing the solubilized catalysts is time-consuming and complicated while generating lots of waste washing water incurring additional cost for biodiesel production. Besides, the NaOH or KOH are sensitive to high content of free fatty acid (FFA), which leading to formation of soap, and complicates the product separation as well as reduces the biodiesel content. Thus, fat or oil with relatively high content of FFA is not employed in conventional approach for biodiesel production in view of its incompatibility to the homogeneous catalysts used. Normally, a two-step reaction is employed to produce the jatropha-based biodiesel where pre-esterification catalyzed by homogeneous acids (H₂SO₄ or H₃PO₄) performed first in order to reduce the content of FFAs. Only then transesterification reaction in following step turns the raw oils to fatty acid methyl esters (FAME) by homogeneous basic NaOH or KOH. To optimally exploit other available oil source having high FFA content such as oil of *Jatropha curcas,* new catalytic system capable of performing transesterification with minimal soap formation and much simpler process is highly appreciated. Preferably, the new catalytic system shall be in the form of heterogeneous catalyst to spare the end products from going through the costly purification step.

### SUMMARY OF THE INVENTION

The present invention aims to provide a method of producing biodiesel from an oil source using a unique heterogeneous binary catalytic system. With the heterogenous catalytic system, the disclosed method is much simplified and cost effective in producing biodiesel.

Another object of the present invention is to provide a cost and time effective method for acquiring biodiesel from a non-edible oil source which is commercially available at lower price compared to its edible counterparts. Consequently, the biodiesel can be produced at relatively lower cost.

Still another object of the present invention is to offer a method of producing biodiesel using an oil source with relatively higher amount of FFA with minimized or even no soap formation.

At least one of the preceding objects is met, in whole or in part, by the present invention, in which one of the embodiments of the present invention includes a method of producing a mixture of methyl ester from an oil source comprising the step of reacting the oil source with an alcohol in the presence of a heterogeneous catalytic system derived from mixed metal oxide of calcium and a second metal. Preferably, the reacting step is performed without neutralizing free fatty acids contained in the oil source in earlier occasion.

In another aspect, the disclosed method may further comprise the step of removing the heterogeneous binary catalytic system via filtration instead of neutralization compared to conventional approach using homogeneous catalysts.

Still another aspect, the oil source suitable to be treated using the disclosed method preferably has relatively higher content of free fatty acids. For example Jatropha curcas oil or used plant oil or used cook oil. Preferably, the oil source has at least 3% by weight of free fatty acids in overall oil composition.. More preferably, the oil source is non-edible oil.

To provide optimal active site to facilitate transesterification, the calcium to the second metal in the mixed metal oxide is preferably in a ratio of 0.25 to 10 : 1 in mole and the the mixed metal oxide can be any one or combination of CaO-ZnO mixed oxide, CaO-CuO mixed oxide, CaO-Cu₂O mixed oxide, CaO-Al₂O₃ mixed oxide, CaO-Ni₂O₃ mixed oxide, CaO-CdO mixed oxide, CaO-Fe₂O₃ mixed oxide, CaO-TiO₂ mixed oxide, CaO-CoO mixed oxide or CaO-Co₂O₃ mixed oxide.

In another aspect, the alcohol to be reacted with the oil source has carbon chain of C1 to C12, either straight or branched, in order to produce fatty acid methyl ester suitable to be employed as fuel source.

### BRIEF DESCRIPTION OF THE DRAWINGS

- Figure 1: is a graph showing XRD patterns for the bulk CaO, ZnO and series of CaO-ZnO mixed metal oxide catalysts;
- Figure 2: is a graph showing TPD-CO₂ for the bulk CaO, ZnO and series of CaO-ZnO mixed metal oxide catalysts;
- Figure 3: shows images of SEM micrographs for the for the different prepared catalysts that (A); ZnO (B); CZ0.5 (C); CZ2 (D); CZ4 (E); CZ6 (F); CZ8 (G) and CZ10 (H)
- Figure 4: is a graph showing correlation between the total basicity and FAME yield of the bulk CaO, ZnO and series of CaO-ZnO mixed metal oxide catalysts;
- Figure 5: shows the effect of reaction time in transesterification reaction where methanol/oil was in a ratio of 20, reaction temperature was 120°C and catalyst amount was3 wt. %;
- Figure 6: shows the effect of methanol/oil molar ratio in transesterification reaction where the reaction time was 3 hours, reaction temperature was 120°C and catalyst amount was 3 wt. %;
- Figure 7: shows the effect of catalyst amount in transesterification reaction where reaction time was 3 hours, reaction temperature was 120°C and methanolo/oil molar ratio was 20;
- Figure 8: shows the effect of reaction temperature where reaction duration was 3 hours, catalyst amount was 3 wt. % and methanol/oil molar ratio was 20;
- Figure 9: (a) is a 2D interaction plot where dotted area equals to experiment FAME yield in which black line representing the catalysts amount of 2 wt. % and dotted line representing the catalysts amount of 4 wt. %, (b) is a 3D surface plot showing interaction effect between reaction time and catalyst amount; transesterification condition where methanol/oil molar ratio was 20 and reaction temperature was 120°C;
- Figure 10: (a) is a 2D interaction plot where dotted area equals to experiment FAME yield in which black line representing the catalysts amount of 2 wt. % and dotted line representing the catalysts amount of 4 wt. %, (b) is a 3D surface plot showing interaction effect between reaction temperature and catalyst amount; transesterification condition where methanol/oil molar ratio was 20 and reaction time was 3 hours; and
- Figure 11: is a graph comparing reusability of the bulk CaO and CZ8 in the transesterification of *Jatropha curcas* oil.

### DETAILED DESCRIPTION OF THE INVENTION

One skilled in the art will readily appreciate that the present invention is well adapted to carry out the objects and obtain the ends and advantages mentioned, as well as those inherent therein. The embodiment describes herein is not intended as limitations on the scope of the invention.

The present invention involves a method of producing a mixture of methyl ester from an oil source comprising the step of reacting the oil source with an alcohol in the presence of a heterogeneous catalytic system derived from mixed metal oxide of calcium and a second metal. The second metal refers to any other metal types besides calcium and it is prepared in the form of oxide to offer the necessary basic property to catalyze transesterification. It was found by the inventors of the disclosed invention that the disclosed heterogeneous catalysts is particularly effective in carrying out transesterification reaction using an oil source with high content of free fatty acids. The solid form catalysts used in the present invention minimizes soap formation in contrast to conventional solvent type catalysts. The heterogeneous base catalyst was designed and replaces homogenous catalyst for the manufacturing of biodiesel via transesterification reaction. Utilization of the heterogeneous catalytic system in the present invention is able to reduce the time consuming and extra purification cost caused by use of homogeneous catalysts. Further, the disclosed method may comprise an additional step of removing the heterogeneous binary catalytic system via filtration. Owing to the solid form of the heterogeneous catalytic system, easy separation of the catalysts from the end product via filtration can be achieved free from the need of washing off the catalysts using fresh water. Moreover, the disclosed method using heterogeneous catalyst can be run either batch of continuous mode giving the producers an option to continue with their current batch reactors or re-change their operations with a packed bed continuous flow reactor operation.

According to the preferred embodiment, the binary system calcium-based mixed metal oxides catalyst is prepared by co-precipitation method of the corresponding mixed metal nitrate solution in the presence of a soluble alkaline carbonate salt solution. The co-precipitated mixed metal oxide is then subjected to calcinations to form the binary catalytic system. The mixed metal oxide can be, but not limited to, CaO-ZnO mixed oxide, CaO-CuO mixed oxide, CaO-Cu₂O mixed oxide, CaO-Al₂O₃ mixed oxide, CaO-Ni₂O₃ mixed oxide, CaO-CdO mixed oxide, CaO-Fe₂O₃ mixed oxide, CaO-TiO₂ mixed oxide, CaO-CoO mixed oxide or CaO-Co₂O₃ mixed oxide. Nevertheless, mixture of both calcium and zinc oxide, CaO-ZnO, is used in the more preferable embodiment. The calcium oxide and the second metal oxide acquired through the co-precipitation method are physically absorbed onto surfaces of one another forming bound oxide powders where the binding does not involve chemical bonds. It is important to be noted that morphology of the mixed oxide obtained from co-precipitation method is different from those acquired via solid state mixing or wet impregnation. Preferably, the catalytic system of the present invention shows high reusability and can be used for few batches of transesterification reaction with low reduced activities. Ratio of second metal oxide and calcium oxide in the catalytic system can significantly influence yield of fatty acid methyl ester. The proportion of the calcium oxide and the second metal oxide co-precipitated and presented in the powder relies on the concentration of their respective salts dissolved in the solution. It is preferable that the calcium to the second metal in the mixed metal oxide is in a ratio of 0.25 to 10: 1 in mole depending on the type of second metal oxide used.

Furthermore, the calcium-based mixed metal oxide catalyst used in the disclosed method is able to tolerate oil source containing high amount of free fatty acids and water, while the oil source herein refers to non-fossil oil such as plant-based oil or animal fat. To attain the object in producing biodiesel with relatively lower price, the oil source used in the present invention is preferably non-edible oil or used edible oil commercially available at lower cost. For example, the oil source is, but not limited to, *Jatropha curcas* oil, used plant oil or used cooking oil. Preferably, the oil source has at least 3 %, and more preferably within 3% to 20%, by weight of free fatty acids and *Jatropha curcas* oil with around 17% of free fatty acids is employed as the oil source in most preferable embodiment. *Jatropha curcas* oil has high content of free fatty acids (FFAs) that it cannot be directly transesterified using homogeneous NaOH or KOH catalyzed process. The considerable amount of free fatty acids (FFAs) in *Jatropha curcas* oil tends to react with these catalyst to form soaps, resulting in serious emulsification which create difficulties in downstream recovery and purification of the biodiesel produced thereof. While, the binary system catalyst used in the disclosed method allows transesterificaton of *Jatropha curcas* oil with an alcohol in one step reaction without requiring pretreatment step for neutralized the free fatty acids content in the oil source.

The disclosed method is capable of achieving high yield of biodiesel (> 80%) via the calcium-based mixed metal oxide catalyzed transesterification reaction. The transesterification activity is believed relying on the basicity of the catalysts. The basicity of the mixed metal oxide was improved by the synergy effect between the CaO and the oxide of the second metal. Moreover, the calcium-based mixed metal oxide used in the present invention shows higher value of basicity than the catalyst of pure calcium oxide.

Pursuant to the preferred embodiment, the alcohol type used in the present invention can be any alcohol with carbon chain of C1 to C12. Yet, methyl, ethyl, isobutyl, or tert-butyl alcohol is preferably used to attain the desired outcome. Further, ratio of the alcohol to the oil source is preferably in the range of 5 to 30 for acquiring the optimal yield. Excessive volume of alcohol is a key factor prompting equilibrium of the transesterification process in the present invention moving towards formation of methyl ester and prohibiting reverse reaction.

In one embodiment, the transesterification process disclosed can be performed at a temperature of 40 to 250°C for a duration of 1 to 8 hours depending on the types of oil source and the yield to be attained. Apart from that, the amount of catalysts used in the reaction may affect the duration and temperature to acquire the desired yield. Preferably, the catalysts used can range from 0.1% to 10% by weight of total volume of the reactants.

More preferably, the reacting step of the present invention is performed without prior neutralization of the free fatty acids contained in the oil source considering the special developed catalytic system employed in the reaction. Nonetheless, pre-treatment such as filtration to remove solid dirt may be performed especially used oil is chosen as the oil source for the reaction.

The following example is intended to further illustrate the invention, without any intent for the invention to be limited to the specific embodiments described therein.

### Example 1

The CaO-ZnO mixed metal oxide catalyst was synthesized by using co-precipitation method. Two aqueous solutions were first prepared. The first solution of 1 M aqueous metallic cation of Ca(NO₃)_{2·}4H₂O and Zn(NO₃)_{2·}6H₂O was dissolved in deionized water. The quantities of both metal nitrate salt were fixed so that the final product has a Ca to Zn ratio equal to 0.25:1, 2:1, 4:1, 6:1, 8:1 and 10:1. The second basic carbonate solution with concentration 2 M was prepared by dissolving appropriate amount of Na₂CO₃ in deionzed water. In the synthesis procedure, the carbonate solution was slowly dropped (10 mL/min) to solution of aqueous metallic cation under vigorous stirring at room temperature. During the gel formation, the mixture was adjusted to pH 10 by 1 M NaOH solution. The mixture was then stirred for 24 h at 60 °C. The solid obtained was then filtered and wash with distilled water until the filtrate was as near pH 7 as possible. The precipitate was then dried in an oven at 100 °C overnight, followed by calcinations at 800 °C for 6 h. The mixed metal oxides obtained were named as CZ0.25, CZ2, CZ4, CZ6, CZ8 and CZ10.

Powder X-ray diffraction analysis was carried out by using a Shimadzu diffractometer model XRD 6000. The diffractometer employing Cu-Kₐ radiation to generate diffraction patterns from powder crystalline samples at ambient temperature. The Cu-Kₐ radiation was generated by Philips glass diffraction X-ray tube broad focus 2.7 kW type. All samples were mounted on samples holder and the basal spacing was determined via powder technique.

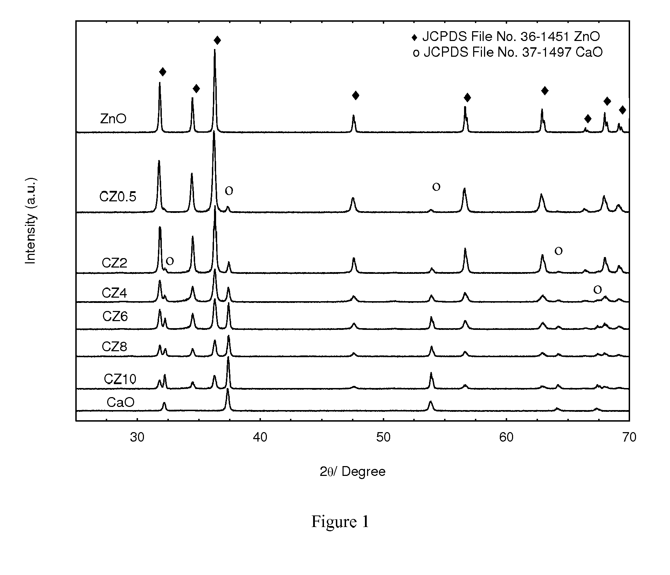

The XRD patterns of bulk CaO, ZnO and binary route CaO-ZnO catalysts with different Ca:Zn ratio shown in Figure 1. The diffraction peaks present in the mixed metal oxide catalysts were corresponded to pure oxide (CaO and ZnO) phases, and no any specific structures attributable to the formation of mixed oxide phase could be detected because they are present as separate oxide clusters in the precipitate particles. The series CaO-ZnO catalysts gave diffraction peaks of CaO at 2θ angles of 37.4° and 53.9° (JCPDS File No. 36-1451) and ZnO at 2θ angles of 31.7°, 34.4° and 36.2° (JCPDS File No. 37-1497) with Wurzite (hexagonal) structure. The diffractograms of CZ0.5 to CZ10 show the increment of intensity of CaO phases when the Ca content was increased. At the same time, the diffraction peaks of ZnO became weaker and weaker as CaO content rising from CZ0.5 to CZ10. The reduction intensities of ZnO diffraction peak are due to the presence of CaO species well dispersed on the surface and thus masking the signal of the ZnO.

### Example 2

The total surface area of the catalysts was measured by the BET (Brunauer-Emmer-Teller) method using nitrogen adsorption at 77 K. The analysis was conducted using Thermo Finnigan Sorptomatic 1900 series nitrogen adsorption/ desorption analyzer.

The BET surface area of CZ0.5, CZ2, CZ4, CZ6, CZ8 and CZ10 catalysts were 10.31, 8.23, 5.46, 5.48, 5.72 and 4.44 m²/ g respectively (Table 1). The results showed that the further incorporation of Ca content on the mixed metal oxide provokes a noteworthy decrease in the corresponding surface area. The decrement of the surface area may due to the over loading of Ca content above saturation point led to masking pore of the catalyst and change of the binary system of mixed metal oxide. Besides, low surface area of CaO-ZnO catalysts is due to high calcination temperature (800 °C) which causing the cluster agglomeration of particle, possibly via a sintering.

The scanning electron microscopy with energy dispersive X-ray detector (SEM-EDX) technique was used to obtain the information of the morphology of the samples. The morphology of the catalysts was carried out using a JEOL scanning electron microscope model JSM-6400. The samples were coated with gold using a Sputter Coater.

The chemical elemental analysis and crystallite size and surface area of the catalysts were summarized in Table 1. The element composition was analysed by using an energy dispersive X-ray detector (EDX) mounted on the microscope. Result of elemental analysis revealed that the ratio of Ca/Zn were reasonable close to the theoretical value. The crystallite sizes of CaO-ZnO catalysts with different ratio were estimated from the XRD patterns using Sherrer's equation. Change in Ca:Zn ratio leads to variation in crystallite sizes of CaO and ZnO in the mixed metal oxide as compared to bulk CaO and ZnO. When the Ca/Zn ratio was increased, the size CaO was increase from 37.94 nm (CZ0.5) to 51.74 nm (CZ10) while the ZnO crystallite sizes slightly change in the range of 34.99 - 38.23 nm for the series of catalysts. The results indicated that crystal sizes of catalysts were in agreement with the line width of the peak in which decrease of FWHM with the increment of the crystallite size. The crystallite sizes of the CaO and ZnO in the mixed metal oxide were depending on the crystallinity of the catalyst. Change of the crystallite sizes may due to the interaction between CaO and ZnO particles.

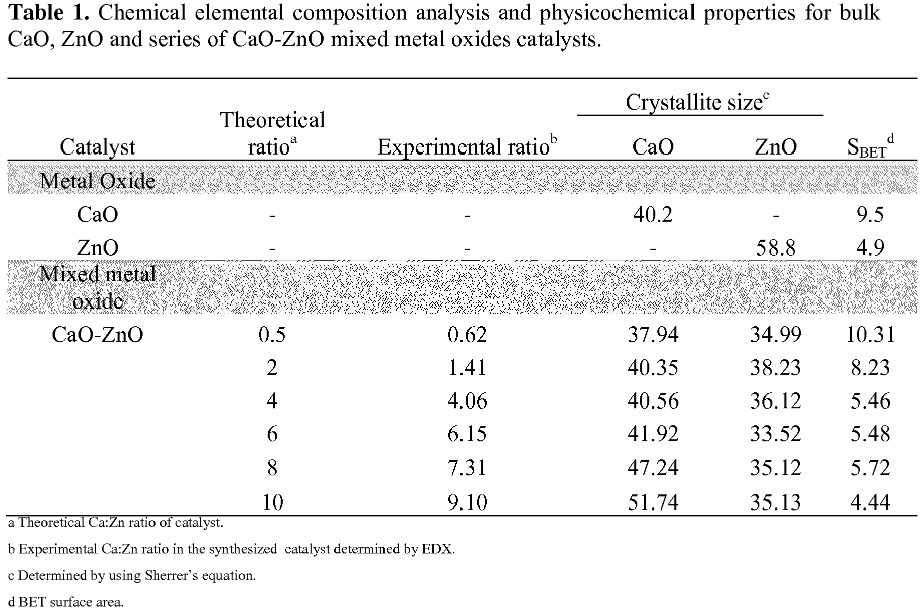

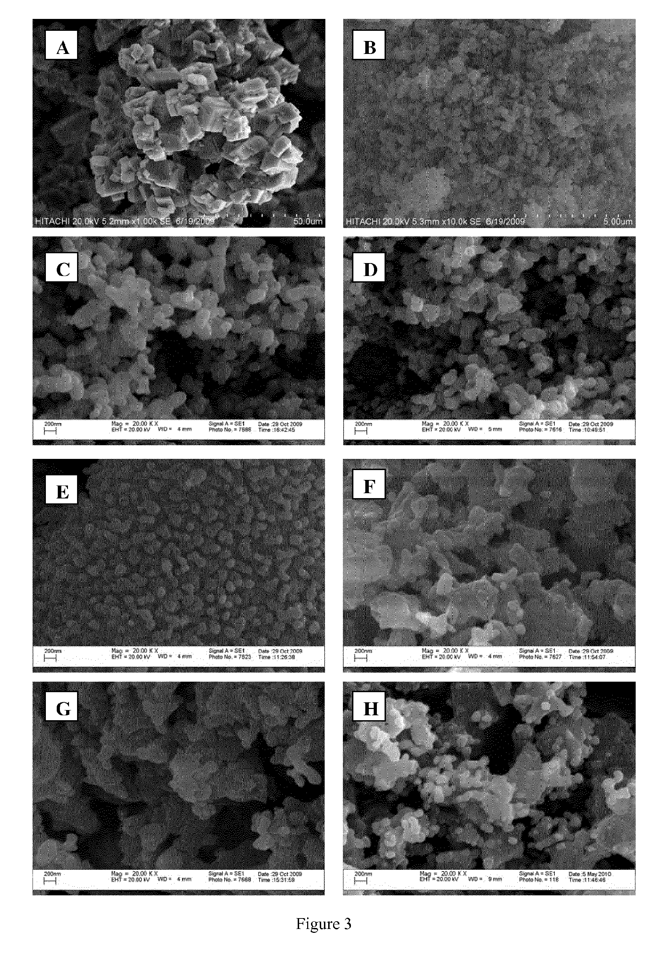

The morphology of catalysts was studied by scanning electron microscopy (SEM). The SEM image of bulk CaO (figure 3A) and ZnO (figure 3B) showed a cluster of well-developed cubic crystal and small round-sahpe particles, respectively. The series CaO-ZnO catalysts gave tiny aggregates particles on the surface of catalysts. It can be observed clearly from SEM images of CZ0.5 (figure 3C) with homogeneous form of round shape particles which have similar structure as bulk ZnO. This is due to the low content of Ca content in the mixed metal oxide. When the Ca content was increased, the catalyst started to agglomerate and two types of oxide particles were present (figure 3D to 3F). The small round-shape particles were corresponded those of ZnO whereas the larger particles size were attributed to those of CaO. Beyond Ca ratio of 4.0, the small particles were merged together and became embedded in the large ones (figure 3G). Moreover, as the Ca content was increased, the particles size of mixed metal oxide became larger due to the coverage of large particles CaO. However, the morphology of the catalyst was change when the Ca content reach ratio of 10, this condition may be due to the over loading of the Ca content led to the structural change (figure 3H).

### Example 3

The basicity of the catalysts were studied by temperature programmed desorption using CO₂ as probe molecule (TPD- CO₂). TPD- CO₂ experiment was performed using a Thermo Finnigan TPDRO 1100 apparatus provided with a thermal conductivity detector. Catalysts (100 mg) were pretreated under a helium stream at 800°C for 30 min (30°C min⁻¹, 30 mL min⁻¹). Then, the temperature was decreased to 50°C and a flow of pure CO₂ (30 mL min⁻¹) was subsequently introduced into the reactor for 1 h. Before the temperature programming up, the sample was flushed with helium at 50°C for 30 min. The analysis of CO₂ desorption was carried out between 100 and 1000 °C under a helium flow (10°C min⁻¹, 30 mL min⁻¹), and detected by thermal conductivity detector (TCD).

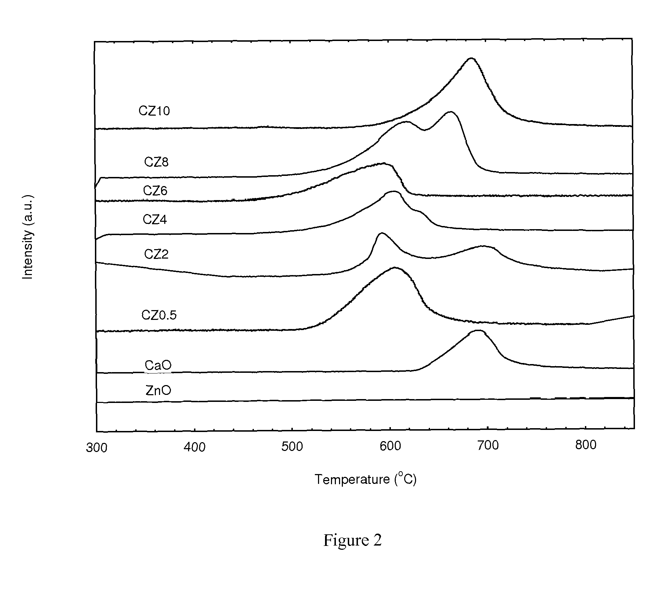

The basic strength and the relative amount of surface basic sites on CaO-ZnO mixed oxides catalysts have been studied by CO₂-TPD. The transesterification activity depends upon the number of basic sites present in a catalyst as well as on their strength. When the CO₂ desorption peak at temperature less than 200 °C, it is attributed to the interaction of CO₂ with weak basic sites in the catalyst. The peak that appeared at 300 - 500 °C can be attributed to moderate basic sites, which are generated due to isolated O²⁻ anions. For the desorption peak that present at a very high temperature (> 500 °C), this might be due to the presence of much stronger basic sites i.e. super basic sites. These strong basic sites probably correspond to isolated O²⁻ anion located in a particular position of the mixed oxide surface. The CO₂-TPD profiles of the catalysts with different Ca/Zn ratio were shown in figure 2. The series of catalysts exhibit an intense and well defined desorption bands at temperature 500 - 800 °C which could be ascribed to the strong interaction of CO₂ with super basic sites of surface catalysts. In point of fact, the ZnO does not adsorb CO₂ in spite of its acid-base character, the strong basic strength of catalysts would point to the existence of a synergy between ZnO and CaO, enhancing the basic character for both CaO and ZnO. In CaO-ZnO system, CaO possess basic sites while ZnO plays an important role by strong interaction between CaO and ZnO leading to the transfer of electrons from metal oxide support interface to ZnO. Therefore, catalysts show the present of super basic strength for the mixed oxide surface.

Calculated from the CO₂-TPD peaks area, the adsorbed CO₂ µmole over per gram of catalysts, which correlated to the surface basicity was listed in Table 2. The total amount of CO₂ uptake on each sample was increased as the increment of Ca:Zn ratio for CZ0.5 to CZ8 but decrease when over loading of Ca content (CZ10). The excessive of Ca content sometime will reduce the basicity instead of being enhanced and this may due to the change of structure or damage of the binary oxide system. From the result, CaO-ZnO binary system showed much higher CO₂ adsorption capacity if compared to pure CaO (290. 42 µ mol of CO₂/g) even though a small content of Ca content loaded for CZ0.5.

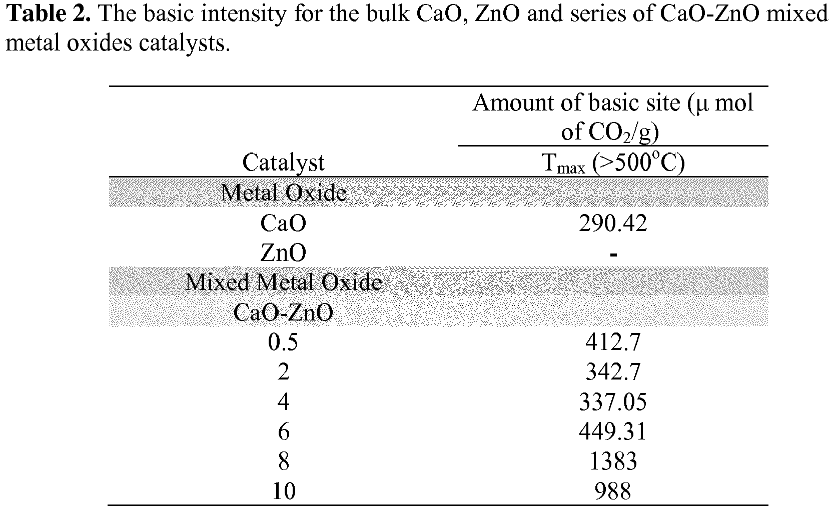

### Example 4

Crude *Jatropha curcas* oil (JCO) was purchased from Bionas Sdn. Bhd. Methanol was commercial grade with 99.5% purity. These liquids were used without further purification. Transesterification was carried out in a BERGHOF high-pressure laboratory reactor In a typical reaction, certain amount of CaO-ZnO catalyst was added to the reaction mixture of 20.0 g of JCO with required volume of methanol. The reactant mixture was heated to different temperature under magnetic agitation with the course of reaction. After the transesterification reaction, the catalyst was separated by centrifuge and the reaction mixture was then loaded into a rotary evaporator to remove excess methanol.

The biodiesel product was analyzed by gas chromatography using Perkin Elmer with FID detector, equipped with a HP INNOWax capillary column. Dichloromethane was used as an internal standard. Injection volume of 0.5 µL was used and the content of methyl ester obtained was calculated using the European regulated procedure EN14103 application note.

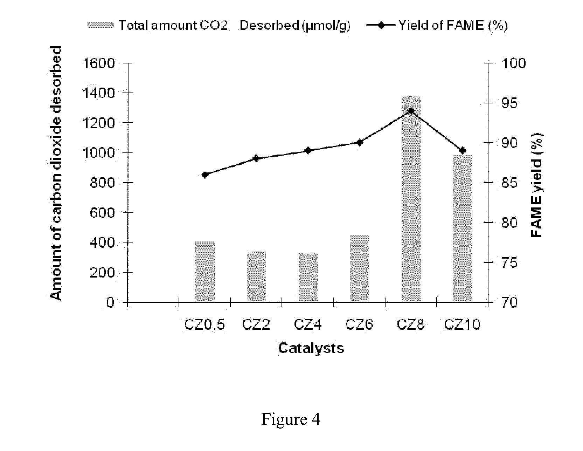

Catalytic transesterification reaction of *Jatropha curcas* oil using synthesized CaO-ZnO catalysts with different of Ca:Zn ratio was performed. The result was presented in figure 4. As seen from this figure, when the Ca content rose from the ratio of 0.5 to 8.0, the FAME yield increased to the maximum value of 94 %. However, as the ratio was above 8.0, the FAME yield was dropped. By drawing on the results, the optimum loading amount of Ca content was ratio 8.0. The excess loading Ca content reduced the interaction between excess CaO with ZnO and thus the stability of the binary oxide system become weak. The leaching of Ca ion from CZ10 increased the difficulty in the separation between FAME and glycerol and reduced the FAME yield product.

As the transesterification activity is depending on the basicity of the catalysts, a good correlation was seen in figure 4, between the observed basicity and catalytic activities of the catalysts. The results suggest that there exists a linear relationship between the total basicity and the yield of FAME from CZ0.5 to CZ10. The observed basicity and transesterification activity of these catalysts demonstrate that the catalyst activity is dependent on their basicity. The high transesterification activity of CZ8 catalyst might be due to the presence of high number of total basic sites compared to its counterparts. Moreover, the catalyst possesses mainly one type of strong basic sites, which could be the reason for the exceptional activity of the catalyst in short reaction times. The high transesterification activity of CZ8 catalyst is due to the manifestation of the dissociation of methanol to RO⁻ and H⁺ on strong basic sites of CaO active phase with high amount of basicity which synergistically enhanced by the optimized interaction between CaO and ZnO. Yield of fatty acid methyl ester via different transesterification reactions experimented using CaO-ZnO of various proportions is shown in table 3 below.

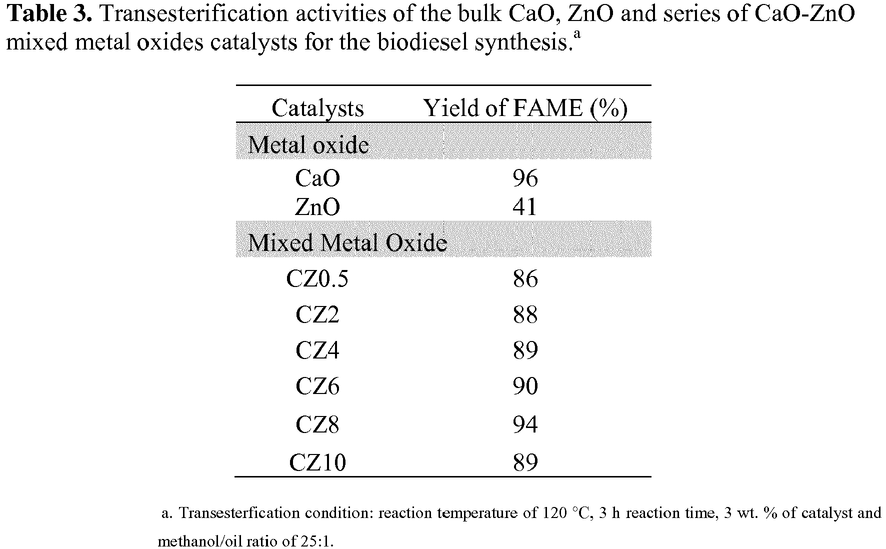

### Example 5

In this study, optimization of operating conditions for the enhancement of biodiesel production using CZ8 mixed oxide solid base catalyst was performed. Central Composite Design (CCD) was used to design the experiments and Response Surface Methodology (RSM) was carried out for process optimization. The reaction temperature, reaction time, methanol/oil molar ratio and catalyst amount were selected as variables to find out their interactive effects.

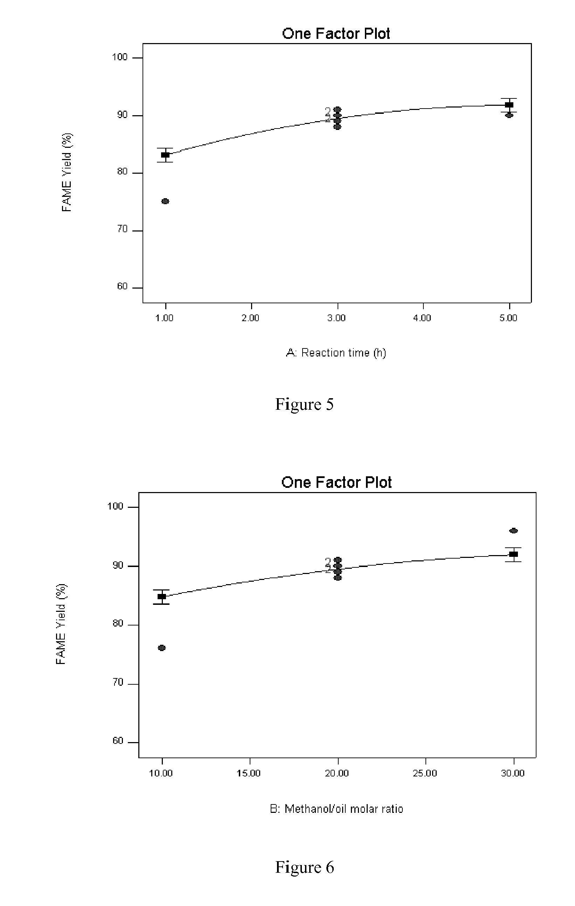

The FAME yield of biodiesel versus reaction time was presented in figure 5. The presence of heterogeneous mass transfer systems and the dispersion of alcohol in the oil lead to the slow reaction within short reaction time. Hence, it is assumed that the catalyst activity was low in the first hour. The FAME yield was increased gradually with further increased of reaction time. The FAME yield was found to be at around 90 % at 3h of reaction time and thereafter remained nearly constant or slightly decreased at 5 h. Excess reaction time will lead to a reduction in the product yield due to the backward reaction of transesterification, resulting in a loss of esters as well as causing more fatty acids to form soaps.

Theoretically, one mole of triglyceride requires three moles of methanol for the transesterification reaction in order to produce fatty acid methyl ester or biodiesel. Triglycerides transesterification is an equilibrium reaction which an excess of alcohol is used to drive the reaction towards completion of ester formation. Refer to the figure 6, the yield of biodiesel was reach > 90 % when the methanol/oil ratio is raised from 10 to 30.

### Example 6

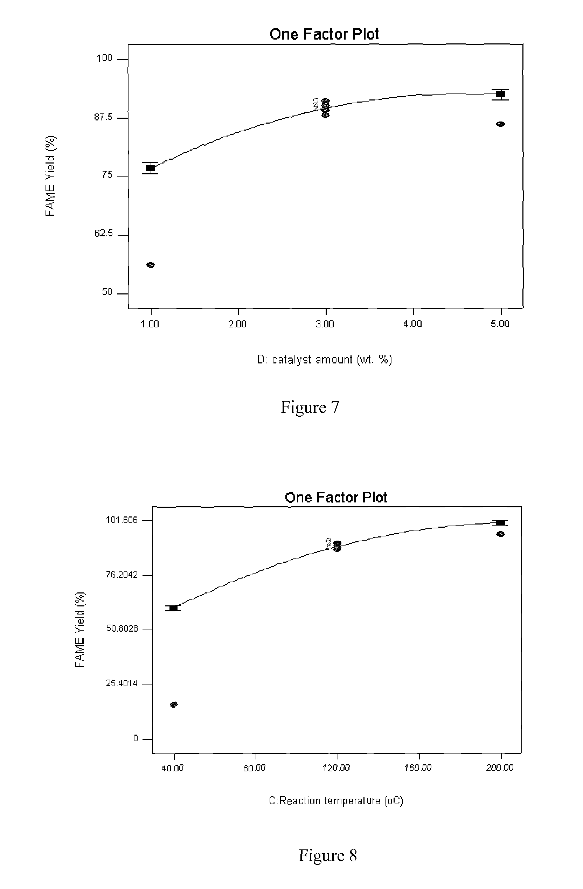

One of the most important factors in affecting the FAME yield in transesterification reaction is catalyst amount. From the reaction profile of figure 7, the FAME yield was increased significantly with the increment of catalyst from 1.0 to 5.0 with referred to the starting oil weight. However, the yield of biodiesel was decreased slightly when the catalyst amount reach 5 wt. %. Excess of catalyst may lead to the resistant of mixing involving reactant, product and solid catalyst. Besides, some of the biodiesel products were absorbed into the catalyst as the dosage of catalyst was too high, and this reduced the biodiesel content.

Among the factors that influence the rate of transesterification reaction, temperature clearly influences the reaction and yield of biodiesel product. A higher reaction temperature can decrease the viscosities of oils and result in an increased reaction rate, and a shortened reaction time. Figure 8 shows that increased of reaction temperature from 40 to 200 °C will significantly bring up the percentage of FAME yield to 99 % within 3 h of reaction time.

### Example 7

The response surface methodology software depicted that reaction time and reaction temperature have a strong interaction effect with the presence of catalyst amount towards the rate of reaction. Reaction time is optimum time for the completion of transesterification reaction. Reaction temperature is able to improve the rate of the reaction within shorter time. Catalyst dosage which consists of active site can enhance the reaction towards the completion of biodiesel synthesis at short time and low reaction temperature.

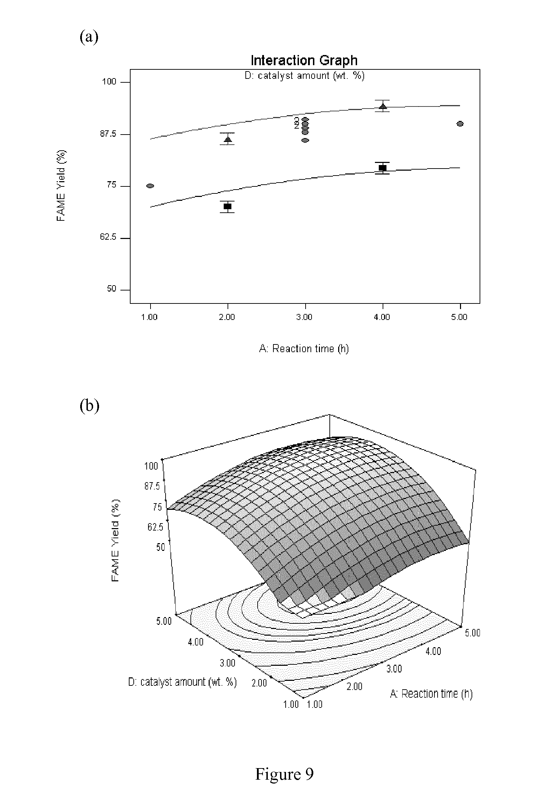

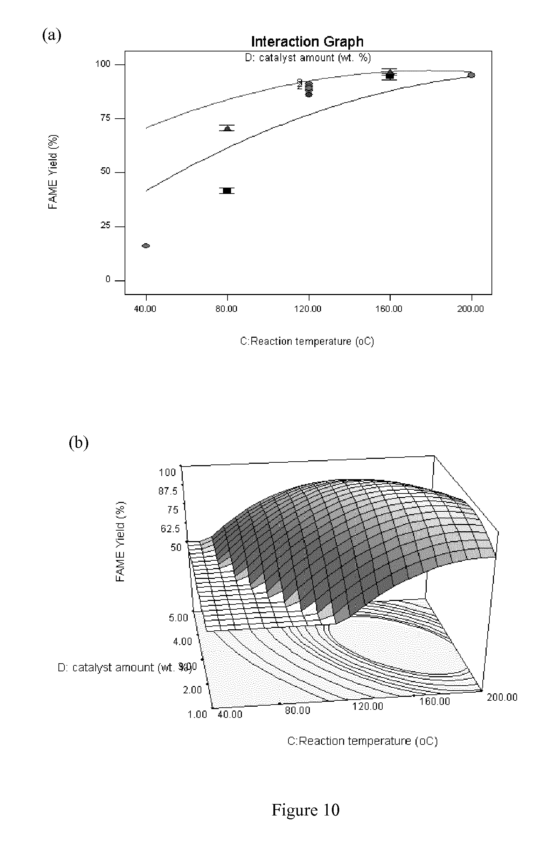

The simultaneous dependence of FAME yield on the reaction time and catalyst amount is shown in figure 9a (2D interaction plot) and figure 9b. (3D surface plot). The other two process parameters: reaction temperature and methanol/oil molar ratio were fixed at 120°C and 20:1, respectively. Figure 10a showed that FAME yield was significantly increase when the catalyst amount was increased from 2 to 4 wt. %. From the 3D surface plot, effect of catalyst amount showed curvilinear effect on FAME yield in which beyond reaction time of 3 h, the FAME yield didn't change significantly or in plateau state. This may due to the reaction reach equilibrium and started to shift the equilibrium to reverse the reaction.

The interaction between reaction temperature and catalyst amount is showed in figure 10. The other two process parameters: reaction time and methanol/oil molar ratio were fixed at 3 h and 20:1, respectively. 2D interaction plot concluded that increment in catalyst amount gave noteworthy increase in FAME yield at low reaction temperature. However, the effect was decreased when the reaction temperature reach 200 °C. It can conclude that temperature is the most influencing factor to initiate the reaction and improving the biodiesel yield no matter how much amount of catalyst was used in the reaction. As shown in 3D surface plot, with low temperature (40 or 80 °C), the increase of catalyst amount did not improve the percentage of FAME yield. However, FAME yield was started to increase when the reaction time reaches 120 °C and above.

### Example 8

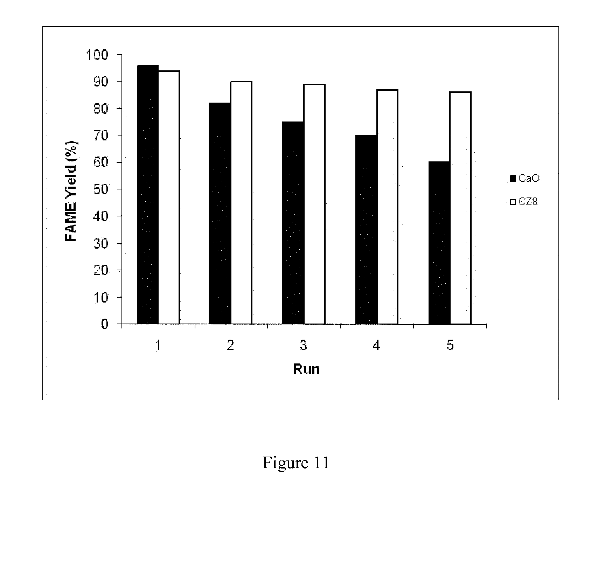

Reusability is one of the important tests required to determine the economical application of CaO-ZnO as heterogeneous catalyst for biodiesel synthesis. To examine the life and reusability of mixed CaO-ZnO catalyst, it was repeatedly served for biodiesel synthesis. The catalyst was filtrated and reused in a new reaction cycle without any post-treatment, such as washing or calcination. Figure 11 shows the results obtained for the usage for the synthesized catalysts of mixed CaO-ZnO oxide (CZ8) and bulk CaO after several cycles. As may be seen, a remarkable reduction in catalytic activity took place in the performed of for CaO catalysts regarding its reusability. The CaO able to maintain the biodiesel yield above 80 % for three recycles only and the activity started to decrease significantly during the fourth recycles. However, CZ8 able to keep the FAME content above 80 % for five recycles. Previous studies have demonstrated that bulk CaO is partially dissolved in the methanolic solution, the lixiviation of calcium active phase causing the existence of ions coming from the catalyst in the biodiesel would involve stages of washing and purification of biodiesel, thus losing one of the most important advantages of a heterogeneous process against a homogeneous one. Without a doubt, there is a strong interaction between active phases CaO and ZnO and thus binary system between active CaO and ZnO able to stabilize CaO preventing its lixiviation to the reaction medium. As a result, the reusability of the mixed CaO-ZnO catalyst is better than that of bulk CaO.

It is to be understood that the present invention may be embodied in other specific forms and is not limited to the sole embodiment described above. However modification and equivalents of the disclosed concepts such as those which readily occur to one skilled in the art are intended to be included within the scope of the claims which are appended thereto.

## Claims

1. A method of producing a mixture of methyl ester from an oil source comprising the step of reacting the oil source with an alcohol in the presence of a heterogeneous catalytic system derived from mixed metal oxide of calcium and a second metal.

2. A method of claim 1 further comprising the step of removing the heterogeneous binary catalytic system via filtration.

3. A method of claim 1 or 2, wherein the oil source has at least 3 % by weight of free fatty acids.

4. A method of claim 1 or 2, wherein the oil source is Jatropha curcas oil or used plant oil.

5. A method of claim 1 or 2, wherein the oil source is non-edible oil.

6. A method of claim 1 or 2, wherein the calcium to the second metal in the mixed metal oxide is in a ratio of 0.25 to 8: 1 in mole.

7. A method of claim 1, wherein the mixed metal oxide is CaO-ZnO mixed oxide, CaO-CuO mixed oxide, CaO-Cu₂O mixed oxide, CaO-Al₂O₃ mixed oxide, CaO-Ni₂O₃ mixed oxide, CaO-CdO mixed oxide, CaO-Fe₂O₃ mixed oxide, CaO-TiO₂ mixed oxide, CaO-CoO mixed oxide or CaO-CO₂0₃ mixed oxide.

8. A method of claim 1, wherein the mixed metal oxide is acquired through co-precipitation followed by calcination.

9. A method of claim 1, wherein the alcohol has carbon chain of C1 to C12.

10. A method of claim 1, wherein the reacting step is performed without neutralizing free fatty acids contained in the oil source.
